# EUROPÄISCHE PATENTSCHRIFT

(11) **EP 1 704 221 B1**
(45) Veröffentlichungstag und Bekanntmachung des Hinweises auf die Patenterteilung: **10.03.2010**
(21) Anmeldenummer: 03818075.8
(22) Anmeldetag: 17.07.2003
(51) Int. Cl.: C12M 1/00, C12M 1/107, A01C 3/02

(54) **BIOGASFERMENTATIONSANLAGE**
BIOGAS FERMENTATION INSTALLATION
DISPOSITIF DE FERMENTATION A BIOGAZ

(43) Veröffentlichungstag der Anmeldung: 27.09.2006
(73) Patentinhaber: Hermann, Gerhard, 4210 Unterweitersdorf (AT); Hanel, Michael, 4310 Rheinfelden (CH)
(72) Erfinder: Hermann, Gerhard, 4210 Unterweitersdorf (AT); Hanel, Michael, 4310 Rheinfelden (CH)
(86) Internationale Anmeldenummer: PCT/EP2003/007773
(87) Internationale Veröffentlichungsnummer: WO 2005/017091

(56) Entgegenhaltungen:
- EP-A- 0 045 114
- EP-A- 1 239 030
- WO-A-95/32158
- WO-A-98/28402
- DE-A- 3 243 103
- DE-A- 3 420 433

## Beschreibung

Die Erfindung bezieht sich auf eine Biogasfermentationsanlage mit wenigstens einem Fermentationsbehälter für auszufaulende biogene Rest- und Abfallstoffe mit den Merkmalen des Oberbegriffes des Anspruches 1.

Bei einer Biogasfermentationsanlage der gleichen Art, gemäß WO 982 84 02 erfolgt die Fermentation der biogenen Abfallstoffe, wie Gülle, in einem Faulbehälter, der quadratisch pyramidenförmig nach unten verjüngend ausgebildet im Boden eingesenkt ist und mit einem Foliengasbehälter dichtend abgedeckt ist, der von einer äußeren pyramidenförmig aufsteigenden Schutzabdeckung überdeckt wird, die von einer Tragkonstruktion gehalten ist.

Nach dem Einleiten von unten wird die biogene Abfallflüssigkeit im Faulbehälter auf Fermentationtemperatur gehalten und zur Verhinderung einer Entmischung umgepumpt und/oder -gerührt.
Das entstehende Gas wird durch eine Waschkolonne geleitet und von Wasser und Schwefelverbindungen befreit und anschließend einer Verbrennungsmaschine einer Wärmekraftkopplung oder einem Heizgerät zur Erzeugung von Strom und Wärme zugeführt die externen und auch internen Verbrauchern zugeführt werden.

Bei den bisher bekannt gewordenen Verfahren und Anordnungen bleibt aber das ausgefaulte Wasser mit Pflanzennährstoffen und der Faulschlamm mit einem erheblichen Wassergehalt zurück, die beide auf landwirtschaftlichen Flächen ausgebracht werden müssen, was wiederum einen bedeutenden Energie- und Arbeitsaufwand ergibt.
Die Konstruktion dieser Biogasfermenteranlage mit Folienabdeckung und die Gestaltung deren Faulbehälters mit einem Flankenwinkel von bis 45° ermöglicht den Austrag von vollständig ausgefaulten Sinkstoffen, die aber noch einen hohen Wassergehalt besitzen und damit ebenfalls eine hohe Transportarbeit erfordern.

Aufgabe der Erfindung ist es, ein Verfahren vorzusehen bei dem die Trennung von Biogas, Feststoffanteil und Fermentationswasser, das heißt von Energie, festen Nährstoffen und Wasser, mit einfachen Mitteln erfolgen kann und die dafür erforderlichen Einrichtungen einfach aufgebaut und benutzbar sind und gleichzeitig eine Hygienisation der festen Nährstoffe und des Fermentationswassers ohne bedeutenden Aufwand auf biologisch freundliche Weise vorgenommen werden kann.

In DE 34 20433 wird eine Zentrifuge einsgesetzt zur Trennung von festen Nährstoffen und Wasser.

Diese Aufgabe wird bei einem Biogasfermenter der eingangs genannten Art mit den Merkmalen des kennzeichnenden Teils des Anspruches 1 und des ersten Vorrichtungsanspruches gelöst.

Die Unteransprüche betreffen besonders vorteilhafte Ausbildungen der Erfindung. Anspruch 1 und die Unteransprüche bilden dabei gleichzeitig einen Teil der Beschreibung der Erfindung.

Durch die Trennung und die getrennte Nachbehandlung des aus dem Biogasfermenter abgezogenen Sedimentats und des Sedimentationswassers wird eine wesentliche Verbesserung der erhaltenen Endprodukte erzielt, indem ein getrocknetes Sedimentat erhalten wird das leicht handhabbar ist und weniger Transportkosten beim Aufbringen auf landwirtschaftliche Flächen erfordert und durch die vorgenommene Hygienisation auch als Handelsprodukt unproblematisch ist. Weiters ermöglicht die Nachbehandlung des Sedimentationswassers in Teichen, bei Abbau und/oder der Aufnahme der enthaltenen Nährstoffe die Gewinnung von Biosubstrat für die Fermentation oder von Tierfutter auf eine einfach handhabare, wenig arbeits- und kapitalaufwendige Weise und das Sedimentationswasser kann überdies als Zusatz für das Biosubstrat bei Einspeisung in den Biogasfermenter Verwendung finden.

Bei zwei nebeneinander angeordneten Biogasfermentern können die Einrichtungen für die Nachbehandlung und zur Befüllung der Biogasfermenter gemeinsam aufgebaut sein und im Betrieb kann ohne Unterbrechung abwechselnd Biogas hergestellt werden.

Um im Biogasfermenter eine konstante Befüllung für den gleichmäßigen Ablauf der Fermentation zu haben, ist es wesentlich, dass die Menge des vorfermentierten Biosubstrates den vorher abgezogenen Mengen an Sedimentat und Sedimentationswasser in etwa entspricht oder dass mit temperiertem Wasser ergänzt wird.

Die Fermentationstemperatur wird der Höhe nach an die Art der Bakterien gebunden sein, deren Zusammensetzung vom Biosubstrat abhängt, doch erfordert ein hoher Umsatz zu Biogas eine entsprechende Fermentationsdauer von ca. 15 Tagen.

Für die Trennung von Sedimentat, den die Schwimmdecken bildenden Schwebstoffen und dem Sedimentationswasser ist eine genügende Ruhezeit von etwa 4 Tagen vorgesehen, nach der der Austrag des Sedimentats und des Sedimentationswassers mit guter Trennwirkung erfolgt.

Bei Anwendung von mehreren, insbesonders von vier oder sechs, Biogasfermentern werden jeweils zwei Biogasfermenter befüllt mit der Fermentation starten, etwa 15 Tage fermentieren, danach je zur Hälfte in einen Sedimentationsfermenter (2) entleert und wieder befüllt werden und nach weiteren etwa 7 Tagen das Sedimentat und das Sedimentationswasser aus dem Sedimentationsfermenter entleert werden, wodurch ein beachtlicher Teil, etwa die Hälfte des Biosubstrats mit den entwickelten Bakterien in der neuen Substratcharge verbleiben, was einen schnelleren Prozessverlauf von Anfang an ermöglicht, die mittlere Verweildauer erhöht und einen vollständigeren Abbau bei größerer Durchsatzleistung ergibt.

Mit der Nachfermentation des Sedimentats wird bei gleichzeitiger Ableitung von noch entstehendem Biogas und von Feuchtigkeit, die in den Prozess zurückgeführt werden, die Hygienisierung durch eine stark erhöhte Temperatur von etwa 70°C vorgenommen.
Für eine gleichmäßige störungsfreie Fermentation ist die Aufbereitung des zugeführten Biosubstrates durch Homogenisation und durch eine Vorfermentation, in Form einer hydrolytischen Behandlung bei erhöhter Temperatur vorgesehen, wobei durch die Anwendung der erhöhten Temperatur von 70° ein Hygenisation bereits in dieser Bearbeitungsstufe erreicht wird.

Für den ungestörte Verlauf der Fermentation und für einen günstigen Wärmehaushalt ist es zweckmäßig, die Temperatur des vor-fermentierten Biosubstrates auf die Fermentationstemperatur des Biogasfermenters einzustellen.

Der Wärmebedarf der Fermenter wird über einen im wesentlichen geschlossenen Wasserkreislauf gedeckt, der bei der Umsetzung zumindest eines Teiles des erzeugten Biogases zur Erzeugung von elektrischer Energie anfällt.

Der Aufbau der Einrichtungen zur Nachbehandlung von Sedimentat und Sedimentationswasser kann in einfachen Behältern oder Teichen erfolgen oder in Betonbecken die entsprechend dem Verfahrensablauf günstig angeordnet und geformt sind und daher auch baulich voneinander getrennt angeordnet sein können, mit Versorgungsleitungen miteinander verbunden und mit einem Minimum an Einbauten von Leitungen und Zugängen und dgl. auskommen, wobei ein zentral liegender Beschickungsschacht die Zu- und Abfuhr von großen Mengen an Fermentationssubstraten durch besonders kurze Wege wesentlich vereinfacht und was wegen der Entleerung des Fermentationswassers zur Vermeidung von Verwirbelungen besonders günstig ist, weil die Entleerung unter Wirkung des hydrostatischen Druckes erfolgen kann.

Bei Anwendung eines Beschickungsschachtes ist die Verwendung einer Förderschnecke zum Abziehen von Fermentat aus dem oder den Biogasreaktor(en) und für das Befüllen mit vorfermentiertem Biosubstrat aus einem mit dem Beschickungsschacht verbundenen oder in diesen integrierten Vorratsspeicher, als Vorfermenter, mit Einspeisung über einen Beschickungsfluter, wegen der kurzen Wege sehr effizient.

Mit der Ausbildung des Vorratsspeichers als gasdichten und temperierbaren Vorfermenter ist es möglich, diesen nach der Homogenisierung als Hydrolysebecken zu betreiben oder zusätzlich ein kleineres Hygienisationsbecken anzuwenden, in dem bei hohen Temperaturen bis 70° eine gute Hygienisierung erreicht wird und anschließend durch Anwendung einer Temperatur des Substrates einen raschen Abbau längerkettiger Kohlenhydrate zu vollziehen und den Biogasfermenter ohne Störung durch Temperaturänderung neu zu befüllen.

Bei Anwendung von Pyramidenfermentern als Biogasfermentern ergibt die Anwendung eines Beschickungsschachtes eine hohe bauliche Vereinfachung und Einsparung. Dabei schließen sich die Wände beiderseits an die Beckenwände der Biogasfermenter an.

Es kann dabei ein großer Teil der Schachtkonstruktion als Wasserpufferspeicher verwendet werden, in dem das für die Prozessführung notwendige Wasser in Rohren zirkuliert.
Im geschlossen Kreislauf kann dadurch kaltes Wasser, das zur Kühlung einer mit Biogas angetriebenen Kraftwärmekopplung nach der Erwärmung zur Temperierung der Biogasfermenter, der Sedimentatfermenterbecken und der Vorratsbecken, wie der Vorfermter oder als Zusatz zum Biosubstrat des Vorfermenters dienen, aber auch zur Temperierung der biologischen Nachklärteiche herangezogen werden und nach Abkühlung wieder rückgeführt werden.

Besonders günstig für den Aufbau einer Schachtkonstruktion des Beschickungsschachtes ist der Aufbau aus Formteilen mit integrierter Verrohrung, bei der die kurzen Wegstrecken entsprechend kurze Längen der Verrohrungen und Leitungen ergeben.
Es ergibt sich daraus baulich ein besonderer Vorteil durch die umfangreiche Vergrößerung des Wasserpufferspeichers für das Warmwasser für den Fermentationsprozess, ohne besonderen zusätzlichen Aufwand und ein hohes gleichmäßig verteiltes Schacht- und Fermenterleergewicht mit der eine hohe Baustabilität der Anlage erreicht wird.

Sofern das hydraulische Gefälle für die Zu- und Abfuhr von Fermentationssubstraten nicht ausreichend ist wird eine zentrale Pumpstation dafür herangezogen.

Die Erzeugung von Biomasse durch Grünpflanzen wird in einfacher arbeitssparender Weise in offenen Teichen vorgenommen und die Biomasse als Tierfutter oder als Biosubstrat für das Fermentationsverfahren verwendet, wobei gleichzeitig gereinigtes Wasser erhalten wird.

Mit einem Druckausgleich der Gasblase, die mit Unter- und Überdruckventilen oder -Klappen erfolgt wird eine Hinterlüftung zwischen Außenhülle und Gasblase überflüssig, wodurch Wärmeverluste durch eine ständige Durchlüftung vermieden werden.

Die für das Verfahren erforderliche Wärmeenergie wird, bei konventionellen Aufbau einer Wärme-Kraft-Kopplungsanlage, aus der Abwärme der Verbrennungsmaschine für die Erzeugung der elektrischen Energie und erforderlichenfalls durch mit Biogas beheizte Wärmegeräte gewonnen, während der Überschuß an Energie als elektrischer Strom oder als Biogas an externe Verbraucher geliefert.

Der Biogasfermenter wird zur Vergrößerung des Nutzeffektes in vorteilhafter Weise als nach unten pyramidenförmig geformten Pyramidenfermenter hergestellt, der in vorteilhafter Weise, als zumindest teilweise in den Boden eintauchendes Fermentationsbecken mit einer nach oben überdachenden pyramidenförmigen Abdeckung versehen ist, unter der eine Gasblase befestigt und im oberen Bereich aufgehängt ist und je nach Füllungsgrad mehr oder weniger aufgebläht lagert.
Die Abdeckung kann von einer Mittelsäule getragen werden oder besonders bei größeren Anlagen von einer Dachstuhlkonstruktion getragen sein.

Bei Anwendungen von Fermentationsbecken mit einem oder zwei lotrechten Wänden, ermöglicht dies einen größeren Fermentationsraum bei sonst gleichen Abmessungen des Fermentationsbeckens, während bei pyramidenförmigen Aufbau die Fermenterwände in Vertiefungen oder auf Aufschüttungen des Bodens von diesem getragen werden, was einen sehr einfachen preisgünstigen Aufbau erlaubt.

Durch die Form der Pyramidenhülle des Biogasfermenters wird der pyroenergetische Einfluß vitalisierend auf die mikrobiellen Abbauprozesse gerichtet, die Abbaurate der zu fermentierenden Biomasse gesteigert und der Brennwert des Biogases und dessen Wasserstoffanteil erhöht. Eine nach unten gerichtete pyramidenförmige oder ähnliche Gestaltung des Biogasfermenterbecken begünstigt die Sedimentation.

Die Erfindung wird anhand der Zeichnung eines Ausführungsbeispieles beschrieben.
Es zeigt:

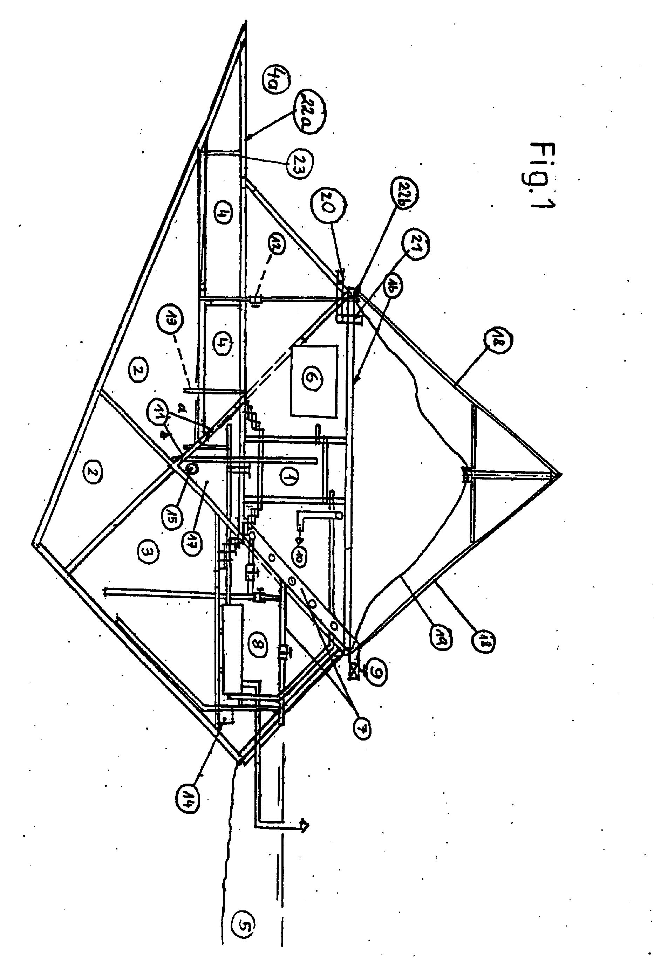

- Fig. 1: eine Einrichtung zur Speicherung und Behandlung einem pyramidenförmigen Biogasfermenter vorgeordnet und zur Nachbehandlung von fermentierten Bio-substrates mit Sedimentationseinrichtung und Sedimentatsfermentationseinrichtung und Speichereinrichtungen nachgeordnet, im Vertikalschnitt, schematisch;

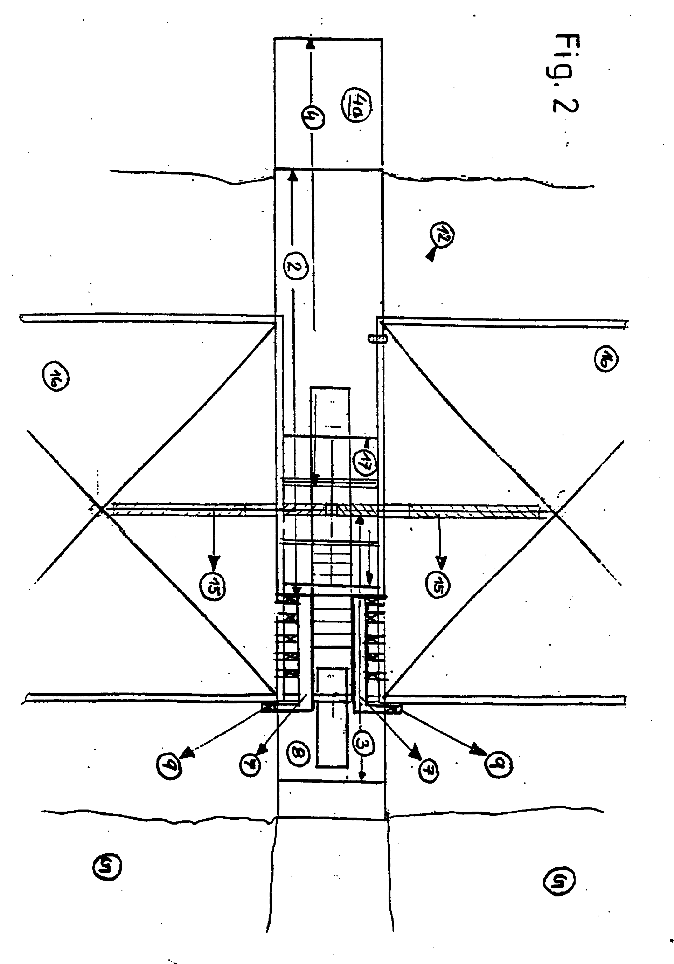

- Fig. 2: eine Draufsicht auf die Einrichtung mit zwei pyramidenförmigen Biogasfermentern, teilweise im Horizontalschnitt, schematisch.

Zwischen zwei, im Abstand parallel zueinander aufgebauten Biogasfermentern 16, deren Fermentationsbecken wie umgekehrte Pyramiden oder Teilpyramiden oder Quadern, mit insbesonders dreieckigen Höhenquerschnitt, in den Erdboden, zumindest teilweise, eingebaut sind, sind die Einrichtungen zur Vorfermentation für zu verarbeitendes Biosubstrat und Einrichtungen zur Nachbehandlung des fermentierten Biosubstrates durch Sedimentation und zur Nachbehandlung und Speicherung von Sedimentat und Sedimentationswasser aus der Fermentation, diesen Abstand ausfüllend angebracht.

Ein Vorratsspeicher 4 der gasdicht verschließbar und mit erwärmten Wasser aus einem Wasserkreislauf temperierbar ist. dient zur Speicherung für zu fermentierendes Biosubstrat und zur Homogenisierung und zur Vorfermentierung durch hydrolytischen Abbau und der Hygienisierung bei etwa 70°.

Dieser kann, wie mit der Teilungswand 23 angedeutet in ein Hygienisationsabteil 4a und in ein größeres Hydrolysebecken 4b aufgeteilt sein, in dem eine Austrittstemperatur von 50°, der Fermentationstemperatur, erreicht wird.

Der Vorratsspeicher 4 erstreckt sich waagrecht in der Höhe eines Beschickungsschachtes 17 für die beiden Biogasfermenter 16 bis an diesen heran, zwischen den beiden Biogasfermentern 16 liegend, während sich darunter liegend und gegen den Vorratsspeicher 4 abgedichtet, ein Sedimentatsfermenter 2 zur Nachfermentation, Ausgasung und Entwässerung und Hygienisation, bei erhöhter Temperatur des Fermentationsprozesses, sich über den Abstand zwischen den beiden umgekehrt pyramidenförmigen oder prismenförmigen Biogasfermentern 16 erstreckend, eingebaut ist.

An den Sedimentatsfermenter 2 schließt an der dem Vorratsspeicher 4 gegenüberliegenden Seite des Beschickungschachtes 17 ein Sedimentationswasserbecken 3 an, über dem ein Maschinenraum 8 mit einer Pumpstation 14 für Zwangsfördervorgänge, wie die Entleerung und Beschickung des Sedimentationswasserbeckens 3 liegt.

Oberhalb des Vorratsspeichers 4 ist ein Raum mit einem Schaltschrank 6 mit Temperaturanzeigen für das Fermentationsheizsystem und der Anzeige für eine Netzeinspeisung, des mit einer Anlage zur Wärmekraftkopplung erzeugten Stromes, angeordnet, bei der das Kühlwasser der Verbrennungsmaschine für dem Wärmekreislauf dient.

Zwischen dem Vorratsspeicher 4 und dem Beschickungsschacht 17 und diesem und dem Sedimentatsfermenter 2 ist ein Beschikkungsfluter 11 angebracht, der je nach Einstellung a oder b oder geschlossen, das vorfermentierte Biosubstrat unter hydrostatischerm Druck aus dem Vorratsspeicher 4 oder das Sedimentat aus dem Biogasfermenter 16 in den in den Beschikkungschacht 17 überführt und von dort ersteres in den Biogasfermenter 16 oder letzteres in den Sedimentatsfermenter 2 geleitet wird.

Aus dem Beschickungsschacht 17 wird das Biosubstrat jeweils einem der beiden Biogasfermenter 16 mit einer Förderschnecke 15 zugeführt oder das Sedimentat entnommen, die in das Becken des Biogasfermenters 17 hineinreicht.

Oberhalb des Beschickungsschachtes 17 ist ein Pufferspeicher 1 mit Wasser, zur Temperierung des Biofermenter 16, des Vorratsspeichers 4 und des Sedimentatsfermenters 2 angeordnet.

Aus dem Sedimentatsfermenter 2 führt eine Gasleitung 12, mit Schieber absperrbar, in den Gasraum des Biogasfermenters 16 zur Überleitung von Biogas und Feuchtigkeit und zur Überprüfung der Entgasung vor Austrag des Sedimentats.

In den Sedimentatsfermenter 2 reicht aus dem Raum oberhalb ein Führungsrohr hinein durch das Sedimentatsproben entnommen werden.

Eine Sedimentations- und Entleerungsprüfeinrichtung 7 für die beiden Biogasfermenter 16 wird von mit deren Wänden ansteigenden Rohren gebildet, die mit den Biogasfermentern 16 durch mehrere in Abständen angeordneten absperrbaren Stichleitungen verbunden sind und am oberen Ende mit Absperrschiebern 9 versehen sind, die der Anlagenfüllstands- und Absetzkontrolle des Sedimentats dienen.
Aus der Gasblase 19 des Biogasfermenters 16 wird das Biogas zur weiteren Verwendung mit der Leitung 10 abgeführt.

Zur Begrenzung von Druckdifferenzen sind übereinander Unterdruckventil 20 und Überdruckventil 21 bzw. -Klappen mit einem Verbindungsrohr eingebaut, das im Freien und in der Gasblase 19 endet und eine dauernde Hinterlüftung zwischen Außenhülle 18 und Gasblase 19 aus wärmetechnischen Gründen entbehrlich macht.

Das Sedimentationswasser wird aus den Biogasfermentern 16 möglichst unter hydrostatischem Druck und aus den Sedimentationswasserbecken 3 mit der Pumpe 14 in Teiche gepumpt, in denen der Entzug der gelösten Pflanzennährstoffe mit Energiepflanzen, wie besonders günstig mit Wasserlinsen erfolgt oder das Wasser wird für Biotope oder Fischteiche oder zur Verdünnung des Biosubstrates im Vorratsspeicher 4 verwendet.

Das aus dem Biogasfermenter 16 abgeleitete Wasser wirkt durch seine pyroenergetische Aufladung revitalisierend auf alle Lebewesen.
Diese Aufladung wird über ein pyroenergetisches Strahlungsfeld erreicht, welches durch die Wechselwirkung zwischen den von der Erde ausgehenden und aus dem Makrokosmos einstrahlenden Energiefeldern erzeugt und über die mit den pyramidenförmige Abdeckung des Biogasfermenters zentriert wird.

### Der Ablauf des Verfahren ist folgender:

Die biogenen Reststoffe werden entsprechend aufbereitet aus dem Vorratsspeicher 4 über einen Beschickungsfluter 11 a,b in den Beschickungsschacht 17 überführt und von einer Beschikkungsschnecke 15 und wahlweise von einer Pumpe 14 im Maschinenraum 8 abwechselnd in einen der beiden Biogasfermenter 16 eingebracht.
Das zu beschickende Substrat soll dabei 15 - 17 % OTS aufweisen.

Der Betrieb der Anlage erfolgt derart, dass ein Biogasfermenter 16 befüllt wird und bei einer Temperatur günstigerweise im thermo- oder mesophilen Bereich ausgast, wobei nach der Befüllung des Biogasfermenters 16 mit Biosubstrat dieses fast vollständig ausgast.

Nach der Zeit des Ausgasens, die ca. 15 Tagen dauert, wird in die Anlage kein weiteres Biosubstrat mehr eingebracht. Es wird ständig für eine gute Durchmischung im Biogasfermenter 16 mittels Rührwerk, Homogenisierungsförder-schnecke und/oder durch Umpumpen über Pumpendüsen gesorgt.
Während der Zeit des Ausgasens des ersten Biogasfermenters 16, wird der zweite Biogasfermenter befüllt.

Nach etwa 15 Tagen ist der Kohlenstoffanteil des Biosubstrates durch die Biomethanisierung im ersten Biogasfermenter nahezu vollständig in Biogas umgewandelt worden und es beginnt der Sedimentations-Prozess. Ohne Rührung oder Beschickung schwimmen im Biogasfermenter die Schwebstoffe zu Schwimmdecken auf und sedimentiert ausgefaulte Biomasse zu Sinkschichten.
Im Zwischenbereich bilden sich biosubstratfreie oder biosubstratarme Wasserschichten.

Nach dem Sedimentationsprozess wird der Biogasfermenter 16 wie folgt entleert:
Bei einem Biomasseanteil der Sinkschichten von über 15% des Biogasfermentervolumens, wird über eine Förderschnecke 15 und über den Beschickungsfluter 11b das Sedimentat, also die Sinkschichten in den Sedimentatsfermenter 2 befördert. In diesem wird das aus dem Biogasfermenter 16 ausgeschiedene Sedimentat wird falls erforderlich bei vorzugsweise ca. 70 Grad Celsius thermisch hygienisiert, über eine Gasleitung 12 restentgast und entwässert.

Das Restgas und die Verdunstungsfeuchtigkeit werden dabei vom Sedimentatsfermenter 2 entsprechend thermodynamischen Grundgesetze in den Biogasfermenter 16 über eine Gasleitung 12 mit Hilfe des dafür ausreichenden Temperaturunterschiedes zwischen dem Sedimentatsfermenter 2 und dem pyramidenförmigen Biogasfermenter 16 in diesen zurückgeführt.

Nach Austragung des Sedimentats in den Sedimentatsfermenter 2, werden die Wasserschichten bis auf 20 % des Biogasfermentervolumens ausgeschieden und wahlweise nach Bedarf in das Sedimentationswasserbecken 3 oder direkt in Teiche 5 geleitet.

Bei Vorhandensein eines höheren Schwimmdeckenanteiles, muss entsprechend mehr Wasservolumen im Biogasfermenter 16 belassen werden.
Nach der Ausscheidung von Sedimentat und Wasser aus dem Biogasfermenter, wird dieser neu befüllt.
Während dieser Zeit, in welcher der erste Biogasfermenter 16 nun zum zweiten Mal befüllt wird, verwandelt sich im zweiten Biogasfermenter der Kohlenstoffanteil des Biosubstrates in Biogas.
Auch im zweiten Biogasfermenter wird die Beschickung nach der Befüllung eingestellt. Nach der Fermentationszeit von ca. 15 Tagen wird ca. 4 Tage sedimentiert, die Sinkschichten auf 15% des Volumens des Biogasfermenters 16 über die Förderschnecke 15 in den Sedimentatsfermenter 2 befördert und der Wassergehalt bis auf 20 % des Fermentervolumens abgezogen.

Danach kann der Biogasfermenter zwei auch wieder befüllt werden.

Auf diese Weise werden zwei Biogasfermenter 16 alternierend zur Energiegewinnung und zur Trennung von Wasser und Nährstoffen eingesetzt, wobei eine humanmedizinisch und veterinärmedizinisch bestmögliche Behandlung des biogenen Abfalls erfolgt, wobei mit diesem Verfahren gleichzeitig die Reinhaltung der Gewässer, die Verbesserung des Bodens und die Geruchsmindernung und die Entlastung der Atmosphäre erreicht wird.

Die mit dem Biosubstrat eingetragenen Bakterienstämme ernähren sich in Symbiose von dem verarbeiteten Rest-Biosubstrat menschlicher Zivilisation und produzieren als wichtiges Stoffwechselprodukt Biogas.

Bei flächendeckender Einführung dieser Verfahrensweise wird einem Naturheilverfahren vergleichbar dazu beitragen, für die Menschen die Belastungen und Gefahren der Energiegewinnung durch lebensfeindliche Techniken zu mindern oder auch ganz zu befreien.

### Bezugszeichen

- 1: Pufferspeicher
- 2: Sedimentatsfermenter
- 3: Sedimentationswasserbecken
- 4: Vorratsspeicher für Gülle und Speisereste o.dgl.
- 4a: Hygienisierungsabteil des Vorratsspeichers 4
- 4b: Hydrolyseabteil des Vorratsspeichers 4
- 5: Teich
- 6: Schaltschrank für Temperaturanzeige von Fermenterheizsystem und Netzeinspeisung
- 7: Sedimentationssichtungs- und Entleerungseinrichtung
- 8: Maschinenraum
- 9: Schieber für die Anlagenfüllstandskontrolle
- 10: Gasleitung vom Biogasfermenter 16 zum Verbraucher, einschließlich Gassicherheitsstrecke
- 11: Beschickungsfluter des Vorratsspeichers in den Beschickungsschacht 17 (a) und Beschickungsfluter von Beschickungsschacht 17 in den Sedimentatsfermenter 2 (b)
- 12: Gasleitung mit Schieber vom Sedimentatsfermenter 2 in den Biogasfermenter 16 zur Entgasung des Sedimentatsfermenters 2 und zur Prüfung vor dessen Entleerung
- 13: Führungsrohr für Sedimentatsprobe
- 14: Pumpe zur Entleerung und Beschickung des Sedimentationswasserbeckens 3 und für die Notkühlung des Maschinenraumes 8
- 15: Förderschnecke des Beschickungsschachtes 17
- 16: Biogasfermenter
- 17: Beschickungsschacht
- 18: Biogasfermenterhülle, Pyramidenhülle
- 19: Gasblase
- 20: Unterdruckventil
- 21: Überdruckventil

- 22: Pyramidenfermenterschachtkonstruktion
- 22a: Schachtsohle auf Höhe des Vorratsspeichers 4
- 22b: Schachtoberkante auf Höhe des oberen Randes des pyramidenförmigen Biogasfermenters 16
- 23: Trennwand zwischen dem Hygienisierungsabteil 4 a und dem Hydrolyseabteil 4b des Vorratsspeichers 4

## Patentansprüche

1. Verfahren zur Trennung von, in einem Biogasfermenter durch Fermentation von Biosubstrat entstandenem Biogas, fermentierter Biomasse und Wasser, **dadurch gekennzeichnet; daß** in einem Biogasfermenter (16) nach der Vorfermentation des eingesetzten Biosubstrates in dem Vorratsspeicher (4), das die ausgegaste fermentierte Biomasse oder Fermentat, durch Beendigen des Umwälzens, einer Absetzphase unterworfen wird, nach der nach dem Absinken der festen Biomasse und nach Aufschwimmen der Schwebstoffe die das Sedimentat bildende Sinkschicht abgezogen wird, die in einem gesonderten geschlossenen Sedimentatsfermenter (2) einer Nachfermentation unterzogen wird, bei der entstehendes Biogas und Feuchtigkeit in den Gasteil des Biogasfermenters (16) geleitet werden und bei der das Sedimentationswasser in einem zweiten Schritt in ein gesondertes Sedimentationswasserbecken (3) oder unmittelbar in Teiche (5) geleitet wird, und ein getrocknetes Sedimentat zurück bleibt in dem Sedimentatsfermenter (2).

2. Verfahren nach Anspruch 1, **dadurch gekennzeichnet, daß** bei zwei parallel nebeneinander angeordneten Biogasfermentern (16) diese abwechselnd mit Biosubstrat gefüllt und nach der Fermentation entleert werden.

3. Verfahren nach einem der vorhergehenden Ansprüche, **dadurch gekennzeichnet, daß** das Absetzen der Sinkstoffe und das Aufschwimmen der Schwebstoffe im Biogasfermenter (16) nach Unterbrechung des Rührvorganges über etwa 4 Tage erfolgt.

4. Verfahren nach einem der vorhergehenden Ansprüche, **dadurch gekennzeichnet, daß** die Nachfermentation des Sedimentats im Sedimentatsfermenter (2) bei stark erhöhter Temperatur, vorzugsweise von etwa 70°C, erfolgt.

5. Verfahren nach einem der vorhergehenden Ansprüche, **dadurch gekennzeichnet, daß** das Biosubstrat vor der Einbringung in die Fermentation in dem Biogasfermenter (16) einer Aufbereitung durch Homogenisierung und weiter einer Vorfermentation, vorzugsweise durch hydrolytische Behandlung bei erhöhter Temperatur, unterworfen wird.

6. Verfahren nach einem der vorhergehenden Ansprüche, **dadurch gekennzeichnet, daß** das Biosubstrat bei der Vorfermentation auf die Temperatur des Biogasfermenters (16) gebracht wird und danach in diesen überführt wird.

7. Vorrichtung zur Ausführung des Verfahren nach einem der vorhergehenden Ansprüche, **dadurch gekennzeichnet, daß** die Einrichtung zur Nachfermentation von Sedimentat im wesentlichen aus einem geschlossenen Sedimentatsfermenter (2) mit einer Gasleitung (12) zur Ableitung des Gases und der Feuchtigkeit in den Gasteil des Biogasfermenters (16) und die Einrichtung für das Sedimentationswasser aus einem Sedimentationswasserbecken (3) und/oder aus wenigstens einem Teich (5) besteht, mit einem zwischen Sedimentatsfermenter (2) und Sedimentationswasserbecken (3) liegenden Beschickungsschacht (17), von dem beide beschickt werden, der Sedimentatsfermenter (2) dabei über einen Beschickungsfluter (11 b). Für die Vorfermentation wird ein Vorratsspeicher (4) benutzt.

8. Vorrichtung zur Ausführung des Verfahren nach einem der vorhergehenden Ansprüche, **dadurch gekennzeichnet, daß** der Beschickungsschacht (17) mit dem oder den Biogasfermenter(n) (16) über eine Förderschnecke (15) zum Abziehen von fermentiertem Biosubstrat und zum Befüllen mit Biosubstrat verbunden ist und über den Beschickungsfluter (11 a) zur Weiterförderung in den Biogasfermenter (16) von einem Vorratsspeicher (4) her beschickt wird.

9. Vorrichtung zur Ausführung des Verfahren nach einem der vorhergehenden Ansprüche, **dadurch gekennzeichnet, daß** der Teich (5) zur Erzeugung von Biomasse eine zur Photosynthese fähige Pflanzenart, wie Wasserlinsen, verwendet wird, wobei die erzeugte Pflanzensubstanz als Tierfutter oder auch als Biosubstrat zur Fermentierung im Biogasfermenter verwendet wird.

## Claims

1. PROCESS for separation of, within a Biofermentation-Plant by fermentation of bio-substrate emerged biogas, fermented biomass, and water, signified by, that within a biogas-fermenter (16)after prefermentation of invested bio-substrate within supply reservoir (4), degassed fermented biomass or fermentate, by stopping circulating, will be subdued to a settling phase, according which after slumping of the solid biomass and after swimming up of suspended matter the sediment forming slump layer will be removed, which will be subdued to a post fermentation within a separated tight sediment-fermenter (2), upon emerging biogas and moisture will be led into the gas section of the biogas-fermenter (16) upon in a second step the sedimentation liquid will be led into a separated sedimentation water basin (3) or directly into ponds (5) whereby a dried sedimentate will remain inside the sediment-fermenter (2).

2. PROCESS according claim 1, signified by, that two parallel to each other situated bio-fermenter (16) will be interchangeably filled and after fermentation emptied.

3. PROCESS according one of preceding claims, signified by, that settling of slump layer and swimming up of suspended matter within biogas-fermenter (16) after interruption the stirring procedure takes place over approximately 4 days.

4. PROCESS according one of preceding claims, signified by, that post fermentation of sedimentate within sediment-fermenter (2) takes place with strongly increased temperature, preferably approximately 70°C.

5. PROCESS according one of preceding claims, signified by, that the bio-substrate before brought to fermentation inside the biogasfermenter (16) will be subdued to preparation through homogenization und additionally through pre-fermentation, preferably by hydrolytic treatment on raised temperature.

6. PROCESS according one of preceding claims, signified by, that the bio-substrate during pre-fermentation will be brought to the temperature of biogas-fermenter (16) and after that will be brought into the same.

7. APPLIANCE for achievement of process according one of preceding claims, signified by, that the appliance for post-fermentation of the sedimentate basically exists in form of a closed sediment-fermenter (2) with a gas pipeline (12) for derivation of gas and moisture within the gas section of biogas-fermenter (16) and an installation for sedimentation water from a sedimentation water basin (3) and/or at least one water pond (5), with one charging hoistway (17) in the middle of sediment-fermenter (2) and sedimentation water basin (3) from which both will be charged, the sediment-fermenter (2) over a charging tube (11 b). For pre fermentation a supply reservoir (4) will be used.

8. APPLIANCE for achievement of process according one of preceding claims, signified by, that the charging hoistway (17) is connected with the biogas-fermenter(s) (16) through a screw conveyer (15) for discharging fermented bio substrate and for charging with bio substrate and is charged through the charging tube (11 a) for on-carriage into the biogas-fermenter (16) from a supply reservoir (4).

9. APPLIANCE for achievement of process according one of preceding claims, signified by, that the pond (5) is used for production of for photosynthesis capable biomass, like duck weed, whereby produced plants biomass can be used as animal fodder or as bio substrate for fermentation inside the biogas-fermenter (16).

## Revendications

1. Procédure de séparation du biogaz, dans une plante de biofermentation par la fermentation du biogaz par le substrat biologique, de biomasse fermentée et de l'eau, signifiée que dans un biogaz-fermenteur (16) après la pré-fermentation de bio-substrat, mis dans le réservoir de stock (4), la biomasse fermentée dégazée ou le ferment, en arrêtant la circulation, seront soumis à une phase d'écoulage, en accordant qu'après la subsidence de la biomasse pleine et après la remonte de la matière en suspension, le sédimentait démoulé qui se trouve dans un fermenteur de sédiment séparé (2), sera soumise à une fermentation ultérieur, dont le biogaz naissant et l'humidité seront menés dans la section de biogaz du biogaz-fermenteur (16) au moment que dans un deuxième pas, l'eau de sédimentation sera mené dans un bassin de sédimentation séparé (3) - ou directement dans des étangs (5) et qu'un sédiment sec demeure au sédiment-fermenteur (2).

2. Procédure selon la revendication 1, marqué du fait que les deux fermentaires de biogaz sont organisés parallèle un à côté de l'autre, et ils sont remplis tour à tour avec le substrat biologique et évacués après la fermentation.

3. Procédure selon des revendications précédents, marqué à travers que la baisse des précipités et la remonte de la matière en suspension au fermentaire de biogaz (16) résulte après une interruption de la procédure de malaxage d'environ 4 jours.

4. Procédure selon des revendications précédents, marqué à travers que la fermentation ensuite du sédimentât au fermentaire de sédimentât (2) aura lieu à une température fortement augmenté, favorisée d'environ 70° dégrée.

5. Procédure selon des revendications précédents, marqué à travers que le substrat de bio, avant le mis en place dans le fermentaire, sera retraité par une homogénéisation et en plus d'une pré-fermentation dans le fermentaire de biogaz (26), favorisé d'une température augmenté.

6. Procédure selon des revendications précédents, marqué à travers que le bio substrat sera mis à la température du biogaz fermentaire lors de la pré-fermentation et sera ensuite traversé dans celuici.

7. Dispositif pour la finition de la procédure selon les revendications précédents, marqué à travers que l'installation pour la fermentation ensuite du sédimentât consiste en principe d'un fermentaire de sédimentas fermée (2) avec la conduite de gaz (12) pour la dérivée du gaz et l'humidité dans la partie de gaz du fermentaire de biogaz (16) et l'installation pour la hydride de sédimentation d'un bassin de la hydride de sédimentation (3) et/ou-bien d'en moins d'un étang (5), avec le puits de participation (17) qui se trouve entre le fermentaire de sédimentas (2) et le bassin de sédimentas par lequel les deux seront alimentés, dont le fermentaire de sédiments (2) par un flûter de participation (11b). Pour la pré-fermentation on utilise un réservoir de stock (4).

8. Dispositif pour la finition de la procédure selon un des revendications précédentes, marqué à travers des demandes précédées, en que le puits de participation (17) qu'avec ou-bien par le biogaz-fermenteur (16) par un transporteur à vis sans fin (15) pour l'affilage du bio-substrat fermenté et pour l'ouillage par le bio-substrat et alimenté par un réservoir de stock (4) par le flûter de participation (11a) pour le réacheminement dans le biogaz-fermenteur (16).

9. Dispositif pour la finition de la procédure selon un des revendications précédentes, marqué à travers que l'étang (5) pour la fabrication de biomasse, des plantes apte pour la photosynthèse comme des lenticules seront employées, tout en que la substance de plante sera employé comme alimentation animale mais aussi comme bio-substrat pour la fermentation au biogaz-fermenteur.
